# EUROPEAN PATENT SPECIFICATION

(11) **EP 2 102 324 B1**
(45) Date of publication and mention of the grant of the patent: **15.06.2011**
(21) Application number: 07854129.9
(22) Date of filing: 17.10.2007
(51) Int. Cl.: C11D 3/00, A61L 12/08

(54) **PACKAGING SOLUTIONS**
VERPACKUNGSLÖSUNGEN
SOLUTIONS D'EMBALLAGE

(30) Priority: 10.11.2006 US 595616
(43) Date of publication of application: 23.09.2009
(73) Proprietor: Bausch & Lomb Incorporated, Rochester, NY 14604-2701 (US)
(72) Inventor: BURKE, Susan E., Batavia, NY 14020 (US); BLACKWELL, Richard I., Webster, NY 14580 (US)
(74) Representative: Glas, Holger
(86) International application number: PCT/US2007/081630
(87) International publication number: WO 2008/060798

(56) References cited:
- WO-A-02/45759
- US-A- 5 298 182
- US-A1- 2003 109 390
- US-A1- 2004 186 028
- US-A1- 2006 229 219

## Description

### BACKGROUND OF THE INVENTION

### 1. Technical Field

The present invention generally relates to packaging solutions for ophthalmic devices such as contact lenses.

### 2. Description of Related Art

Blister-packs and glass vials are typically used to individually package each soft contact lens for sale to a customer. Saline or deionized water is commonly used to store the lens in the blister-packs, as mentioned in various patents related to the packaging or manufacturing of contact lenses. Because lens material may tend to stick to itself and to the lens package, packaging solutions for blister-packs have sometimes been formulated to reduce or eliminate lens folding and sticking. For this reason, polyvinyl alcohol (PVA) has been used in contact lens packaging solutions. Additionally, U.S. Patent No. 6,440,366 discloses contact lens packaging solutions comprising polyethylene oxide (PEO)/polypropylene oxide (PPO) block copolymers, especially poloxamers or poloxamines.

US 2004/0186028 A1 discloses an ophthalmic solution for soft contact lenses for controlled release of polyethers into an eye's tear film. It further discloses the use of cationic polyelectrolytes for controlling the swelling of hydrogel contact lenses typically caused by the absorption of high concentrations of polyethers.

US 2003/0109390 A1 discloses new and improved solutions for packaging contact lenses and to methods for treating contact lenses with such solutions to improve the comfort of the lenses during wear. In particular, it discloses packaging solutions comprising certain non-ionic surfactants containing a poly(oxyalkylene) copolymer and having a molecular weight of 4000 to 30,000.

US 2006/0229219 A1 discloses a buffering system having a pKa which may be selected based on the environment in which the solution is designed to be used. Solutions containing such buffering systems may include borate-polyol complexes as the primary buffering agents, and may include one or more of the following: an aqueous liquid medium; an antimicrobial component; a surfactant component; a viscosity-inducing component; and/or a tonicity component.

It has been stated that if a lens is thoroughly cleaned before insertion, lacrimal fluid can adequately wet the lens. Furthermore, the difficulties of adding a surfactant to a packaging solution, including the possibility of lowering shelf-life and/or adverse reactions during heat sterilization, have further limited the use of surfactants in a packaging solution for the purpose of providing any possible or marginal effect on lens comfort. It is only after a lens has been worn, when proteins or other deposits have formed on the surface of the lens, that surfactants have been used in standard lens-care solutions.

It is highly desirable that contact lenses be as comfortable as possible for wearers. Manufacturers of contact lenses are continually working to improve the comfort of the lenses. Nevertheless, many people who wear contact lenses still experience dryness or eye irritation throughout the day and particularly towards the end of the day. An insufficiently wetted lens at any point in time will cause significant discomfort to the lens wearer. Although wetting drops can be used as needed to alleviate such discomfort, it would certainly be desirable if such discomfort did not arise in the first place.

Accordingly, it would be desirable to provide an improved packaging system for ophthalmic lenses such that the lenses would be comfortable to wear in actual use and allow for extended wear of the lenses without irritation or other adverse effects to the cornea.

### SUMMARY OF THE INVENTION

In accordance with one embodiment of the present invention, a method of preparing a package comprising a storable, sterile ophthalmic device is provided comprising:
(a) immersing an ophthalmic device in a solution comprising a nonionic, nonpolymeric polyol and a nonionic, polymeric conditioning agent, wherein the solution has an osmolality of at least about 200 mOsm/kg and a pH in the range of about 6 to about 8, and wherein the concentration of the nonionic, polymeric conditioning agent is about 0.01 to about 10% w/w;
(b) packaging the solution and the device in a manner preventing contamination of the device by microorganisms; and
(c) sterilizing the packaged solution and device.

In accordance with a second embodiment of the present invention, a method for packaging and storing a contact lens is provided comprising, prior to delivery of the contact lens to the customer-wearer, immersing the contact lens in an aqueous contact lens packing solution inside a package and heat sterilizing the solution, wherein the contact lens packing solution comprising a nonionic, nonpolymeric polyol and a nonionic polymeric conditioning agent, wherein the solution has an osmolality of at least about 200 mOsm/kg and a pH of about 6 to about 8, and wherein the concentration of the nonionic, polymeric conditioning agent is about 0.01 to about 10% w/w.

In accordance with a third embodiment of the present invention, a packaging system for the storage of an ophthalmic device is provided comprising a sealed container containing one or more unused ophthalmic devices immersed in an aqueous packaging solution comprising a nonionic, nonpolymeric polyol and a nonionic polymeric conditioning agent, wherein the solution has an osmolality of at least about 200 mOsm/kg, a pH of about 6 to about 8, and wherein the concentration of the nonionic, polymeric conditioning agent is about 0.01 to about 10% w/w and is heat sterilized.

In accordance with a fourth embodiment of the present invention, a packaging system for the storage of an ophthalmic device is provided comprising:
(a) a solution comprising a nonionic, nonpolymeric polyol and a nonionic polymeric conditioning agent, wherein the solution has an osmolality of at least about 200 mOsm/kg and a pH in the range of about 6 to about 8, and wherein the concentration of the nonionic, polymeric conditioning agent is about 0.01 to about 10% w/w;
(b) at least one ophthalmic device; and
(c) a container for holding the solution and ophthalmic device sufficient to preserve the sterility of the solution and ophthalmic device, wherein the solution does not contain an effective disinfecting amount of a disinfecting agent.

By combining a nonionic, nonpolymeric polyol and a nonionic polymeric conditioning agent to form a packaging solution, it has been discovered that the combination lowers the surface friction of a contact lens over that of a lens stored in a buffered saline solution or a lens stored in a buffered saline solution containing a conditioning polymer, thus increasing the lubricity of the lens surface. Thus, the lens will be more comfortable to wear in actual use and would allow for the extended wear of the lens without irritation or other adverse effects to the cornea.

### DETAILED DESCRIPTION OF THE PREFERRED EMBODIMENTS

The present invention provides a packaging system for the storage of ophthalmic devices intended for direct contact with body tissue or body fluid. As used herein, the term "ophthalmic device" refers to devices that reside in or on the eye. These lenses can provide optical correction, wound care, drug delivery, diagnostic functionality or cosmetic enhancement or effect or a combination of these properties. Representative examples of such devices include, but are not limited to, soft contact lenses, e.g., a soft, hydrogel lens; soft, non-hydrogel lens and the like, hard contact lenses, e.g., a hard, gas permeable lens material and the like, intraocular lenses, overlay lenses, ocular inserts, optical inserts and the like. As is understood by one skilled in the art, a lens is considered to be "soft" if it can be folded back upon itself without breaking. Any material known to produce an ophthalmic device including a contact lens can be used herein.

It is particularly useful to employ biocompatible materials herein including both soft and rigid materials commonly used for ophthalmic lenses, including contact lenses. The preferred substrates are hydrogel materials, including silicone hydrogel materials. Particularly preferred materials include vinyl functionalized polydimethylsiloxanes copolymerized with hydrophilic monomers as well as fluorinated methacrylates and methacrylate functionalized fluorinated polyethylene oxides copolymerized with hydrophilic monomers. Representative examples of substrate materials for use herein include those disclosed in U.S. Patent Nos. 5,310,779; 5,387,662; 5,449,729; 5,512,205; 5,610,252; 5,616,757; 5,708,094; 5,710,302; 5,714,557 and 5,908,906, the contents of which are incorporated by reference herein.

A wide variety of materials can be used herein, and silicone hydrogel contact lens materials are particularly preferred. Hydrogels in general are a well-known class of materials that comprise hydrated, cross-linked polymeric systems containing water in an equilibrium state. Silicone hydrogels generally have a water content greater than about 5 weight percent and more commonly between about 10 to about 80 weight percent. Such materials are usually prepared by polymerizing a mixture containing at least one silicone-containing monomer and at least one hydrophilic monomer. Typically, either the silicone-containing monomer or the hydrophilic monomer functions as a crosslinking agent (a crosslinker being defined as a monomer having multiple polymerizable functionalities) or a separate crosslinker may be employed. Applicable silicone-containing monomeric units for use in the formation of silicone hydrogels are well known in the art and numerous examples are provided in U.S. Patent Nos. 4,136,250; 4,153,641; 4,740,533; 5,034,461; 5,070,215; 5,260,000; 5,310,779, and 5,358,995.

Representative examples of applicable silicone-containing monomeric units include bulky polysiloxanylalkyl(meth)acrylic monomers. An example of a bulky polysiloxanylalkyl(meth)acrylic monomer is represented by the structure of Formula I: 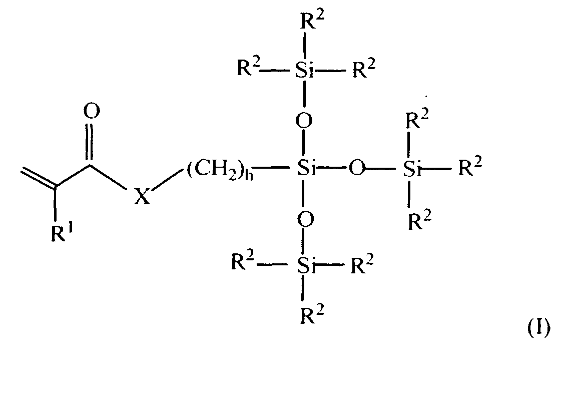 wherein X denotes -O- or -nor-, each R¹ independently denotes hydrogen or methyl; each R² independently denotes a lower alkyl radical, phenyl radical or a group represented by 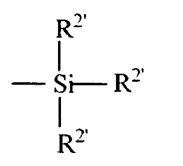 wherein each R² independently denotes a lower alkyl or phenyl radical; and h is 1 to 10. Examples of bulky monomers are methacryloxypropyl tris(trimethyl-siloxy)silane or tris(trimethylsiloxy)silylpropyl methacrylate, sometimes referred to as TRIS and tris(trimethylsiloxy)silylpropyl vinyl carbamate, sometimes referred to as TRIS-VC and the like.

Such bulky monomers may be copolymerized with a silicone macromonomer, which is a poly(organosiloxane) capped with an unsaturated group at two or more ends of the molecule. U.S. Patent No. 4,153,641 discloses, for example, various unsaturated groups such as acryloxy or methacryloxy groups.

Another class of representative silicone-containing monomers includes, but is not limited to, silicone-containing vinyl carbonate or vinyl carbamate monomers such as, for example, 1,3-bis[4-vinyloxycarbonyloxy)but-1-yl]tetramethyl-disiloxane; 3-(trimethylsilyl)propyl vinyl carbonate; 3-(vinyloxycarbonylthio)propyl-[tris(dimethylsiloxy)silane]; 3-[tris(trimethylsiloxy)silyl]propyl vinyl carbamate; 3-[tris(trimethylsiloxy)silyl]propyl allyl carbamate; 3-[tris(trimethylsiloxy)silyl]propyl vinyl carbonate; t-butyldimethylsiloxyethyl vinyl carbonate; trimethylsilylethyl vinyl carbonate; trimethylsilylmethyl vinyl carbonate and the like and mixtures thereof.

Another class of silicone-containing monomers includes polyurethane-polysiloxane macromonomers (also sometimes referred to as prepolymers), which may have hard-soft-hard blocks like traditional urethane elastomers. They may be end-capped with a hydrophilic monomer such as 2-hydroxyethyl methacrylate (HEMA). Examples of such silicone urethanes are disclosed in a variety or publications, including U.S. Patent No. 6,858,218 and PCT Published Application No. WO 96/31792, which disclosures are hereby incorporated by reference in their entirety. Further examples of silicone urethane monomers are represented by Formulae II and III:

E(*D*A*D*G)₃ *D*A*D*E'; (II)

or

E(*D*G*D*A)ₐ *D*A*D*E'; (III)

or
wherein:
D independently denotes an alkyl diradical, an alkyl cycloalkyl diradical, a cycloalkyl diradical, an aryl diradical or an alkylaryl diradical having 6 to about 30 carbon atoms;
G independently denotes an alkyl diradical, a cycloalkyl diradical, an alkyl cycloalkyl diradical, an aryl diradical or an alkylaryl diradical having 1 to about 40 carbon atoms and which may contain ether, thio or amine linkages in the main chain;
* denotes a urethane or ureido linkage;
a is at least 1;
A independently denotes a divalent polymeric radical of Formula IV: 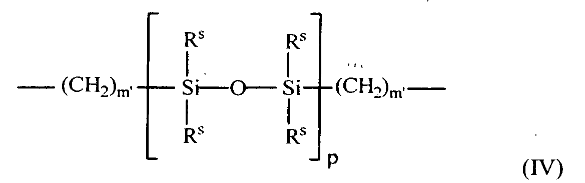 wherein each R^{s} independently denotes an alkyl or fluoro-substituted alkyl group having 1 to about 10 carbon atoms which may contain ether linkages between the carbon atoms; m' is at least 1; and p is a number that provides a moiety weight of about 400 to about 10,000;
each of E and E' independently denotes a polymerizable unsaturated organic radical represented by Formula V: 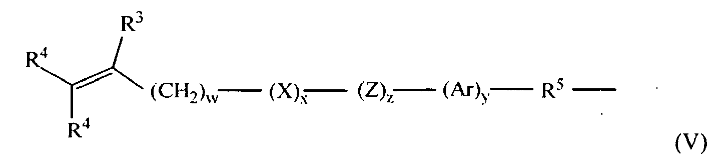 wherein: R³ is hydrogen or methyl;
   R⁴ is hydrogen, an alkyl radical having I to 6 carbon atoms, or a -CO-Y-R⁶ radical wherein Y is -O-, -S- or -NH-;
   R⁵ is a divalent alkylene radical having 1 to about 10 carbon atoms;
   R⁶ is a alkyl radical having 1 to about 12 carbon atoms;
   X denotes -CO- or -OCO-;
   Z denotes -O- or -NH-;
   Ar denotes an aromatic radical having about 6 to about 30 carbon atoms;
   w is 0 to 6; x is 0 or 1, y is 0 or 1, and z is 0 or 1.

A specific example of a silicone-containing urethane monomer is represented by Formula VI: 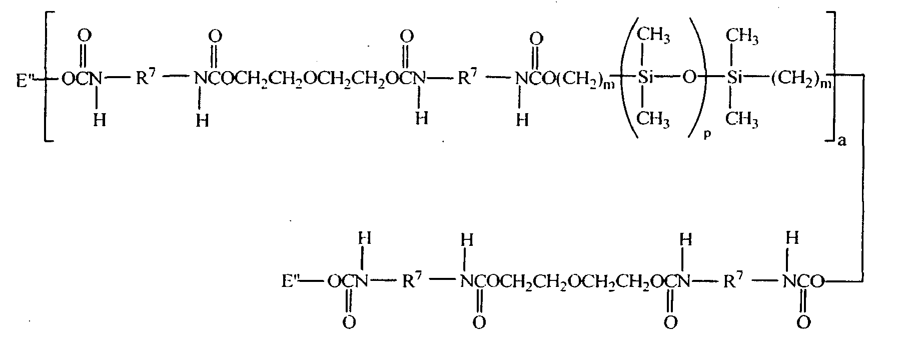 wherein m is at least 1 and is preferably 3 or 4, a is at least 1 and preferably is 1, p is a number which provides a moiety weight of about 400 to about 10,000 and is preferably at least about 30, R⁷ is a diradical of a diisocyanate after removal of the isocyanate group, such as the diradical of isophorone diisocyanate, and each E" is a group represented by:

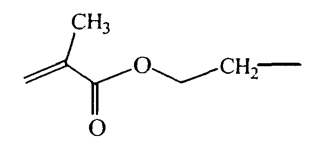

In another embodiment of the present invention, a silicone hydrogel material comprises (in bulk, that is, in the monomer mixture that is copolymerized) about 5 to about 50 percent, and preferably about 10 to about 25, by weight of one or more silicone macromonomers, about 5 to about 75 percent, and preferably about 30 to about 60 percent, by weight of one or more polysiloxanylalkyl (meth)acrylic monomers, and about 10 to about 50 percent, and preferably about 20 to about 40 percent, by weight of a hydrophilic monomer. In general, the silicone macromonomer is a poly(organosiloxane) capped with an unsaturated group at two or more ends of the molecule. In addition to the end groups in the above structural formulas, U.S. Patent No. 4,153,641 discloses additional unsaturated groups, including acryloxy or methacryloxy. Fumarate-containing materials such as those disclosed in U.S. Patent Nos. 5,310,779; 5,449,729 and 5,512,205 are also useful substrates in accordance with the invention. Preferably, the silane macromonomer is a silicone-containing vinyl carbonate or vinyl carbamate or a polyurethane-polysiloxane having one or more hard-soft-hard blocks and end-capped with a hydrophilic monomer.

Suitable hydrophilic monomers include amides such as dimethylacrylamide and dimethylmethacrylamide, cyclic lactams such as n-vinyl-2-pyrrolidone and poly(alkene glycols) functionalized with polymerizable groups. Examples of useful functionalized poly(alkene glycols) include poly(diethylene glycols) of varying chain length containing monomethacrylate or dimethacrylate end caps. In a preferred embodiment, the poly(alkene glycol) polymer contains at least two alkene glycol monomeric units. Still further examples are the hydrophilic vinyl carbonate or vinyl carbamate monomers disclosed in U.S. Patent No. 5,070,215, and the hydrophilic oxazolone monomers disclosed in U.S. Patent No. 4,910,277. Other suitable hydrophilic monomers will be apparent to one skilled in the art.

The above silicone materials are merely exemplary, and other materials for use as substrates that can benefit by being packaged in the packaging solution according to the present invention and have been disclosed in various publications and are being continuously developed for use in contact lenses and other medical devices can also be used. For example, an ophthalmic lens for use herein can be a cationic lens such as a cationic contact lens or fluorinated silicone-containing monomers. Such monomers have been used in the formation of fluorosilicone hydrogels to reduce the accumulation of deposits on contact lenses made therefrom, as disclosed in, for example, U.S. Patent Nos. 4,954,587; 5,010,141 and 5,079,319. The use of silicone-containing monomers having certain fluorinated side groups, i.e., -(CF₂)-H, have been found to improve compatibility between the hydrophilic and silicone-containing monomeric units. See, e.g., U.S. Patent Nos. 5,321,108 and 5,387,662.

Ophthalmic devices such as contact lenses for application of the present invention can be manufactured employing various conventional techniques, to yield a shaped article having the desired posterior and anterior lens surfaces. Spincasting methods are disclosed in U.S. Patent Nos. 3,408,429 and 3,660,545; static casting methods are disclosed in U.S. Patent Nos. 4,113,224, 4,197,266, and 5,271,875.

The present invention is especially advantageous with respect to a contact lens for extended-wear or specialty uses. Extended lenses are lenses capable of being worn overnight, preferably capable of being worn for at least one week, most preferably capable of wear for a continuous period of one week to one month. By "capable" is meant lenses approved by one or more governmental regulatory authorities for such consumer use, for example, the U.S. Food & Drug Administration (USFDA) in the U.S. or its equivalent in other countries.

Extended-wear lenses require relatively high oxygen permeability. The oxygen-permeability is the rate at which oxygen will pass through a material. The oxygen-permeability (Dk) of a lens material does not depend on lens thickness. Oxygen permeability is measured in terms of barrers.

On the other hand, the oxygen transmissibility of a lens, as used herein, is the rate at which oxygen will pass through a specific lens. Oxygen transmissibility, Dk/t, is conventionally expressed in units of barrers/mm, where t is the average thickness of the material (in units of mm) over the area being measured. For example, a lens having a Dk of about 90 barrers (oxygen-permeability barrers) and a thickness of about 90 microns (about 0.090 mm) would have a Dk/t of about 100 barrers/mm (oxygen transmissibility barrers/mm).

Next, the lens will be immersed in a packaging solution and stored in a packaging system according to the present invention. Generally, a packaging system for the storage of an ophthalmic lens according to the present invention includes at least a sealed container containing one or more unused ophthalmic lenses immersed in an aqueous lens packaging solution. Preferably, the sealed container is a hermetically sealed blister-pack, in which a concave well containing a contact lens is covered by a metal or plastic sheet adapted for peeling in order to open the blister-pack. The sealed container may be any suitable generally inert packaging material providing a reasonable degree of protection to the lens, preferably a plastic material such as polyalkylene, PVC, polyamide, and the like.

Any suitable nonionic polymeric conditioning agent component may be employed in accordance with the present invention provided that it functions as described herein and has no substantial detrimental effect on the contact lens being stored or on the wearer of the contact lens. This component is ophthalmically acceptable at the concentrations used. Particularly useful components are those, which are water soluble, for example, soluble at the concentrations used in the presently useful liquid aqueous media.

These compounds condition the lens by providing one or more of the following attributes: increased viscosity for increase retention time on the lens; enhanced wetting of the lens surface; decreased surface friction; or enhanced comfort of a contact lens by forming a cushioning film over the lens surface.

A class of nonionic, polymeric conditioning agents includes nonionic polysaccharides. Representative examples of suitable components for use herein include, but are not limited to, methylcellulose; hydroxyethylcellulose; hydroxypropylcellulose; hydroxypropylmethylcellulose; and methylhydroxyethyl starches.

Another class of nonionic, polymeric conditioning agents includes polyvinylalcohols and polyvinylpyrrolidones.

Another class of nonionic, polymeric conditioning agents includes polymers of PEO, including PEO homopolymers, and block copolymers of PEO and PPO. This class includes poloxamers and poloxamines, including those disclosed in U.S. Patent No. 6,440,366.

The above classes of nonionic, polymeric conditioning agents are intended for illustrative purposes only and not to limit the scope of the present invention. Such polymers are known to those of skill in the art.

Generally, the average molecular weight of nonionic, polymeric conditioning agent is a minimum of about 1 kDa and a maximum of about 700 kDa, more preferably, about 5 kDa to 500 kDa.

The amount of nonionic, polymeric conditioning agent employed is that amount effective to improve the surface properties of the ophthalmic device when combined with a nonionic, nonpolymeric polyol. Preferably the nonionic, polymeric conditioning agent is present in the packaging solution of the invention in an amount of at least 0.01% w/v. The specific amount of such component used can vary widely depending on a number of factors, for example, the specific polymeric component and nonionic polyol being employed. The concentration of the nonionic, polymeric conditioning agent is from about 0.01 to about 10 % w/w and preferably from about 0.5 to about 1.5 % w/w.

In one embodiment, the nonionic, nonpolymeric polyol for use herein can be a nonionic polyol containing 2 to about 12 carbon atoms and preferably 2 to 4 carbon atoms and from 2 to 8 hydroxyl groups. Representative examples of such nonionic polyols include glycerin, ethylene glycol, propylene glycol, sorbitol, manitol, monosaccarides, disaccharides such as trehalose, and the like and mixtures thereof. In one embodiment, the nonionic polyol can be glycerin, ethylene glycol, sorbitol, mannitol, monosaccharides and mixtures thereof.

The amount of the nonionic, nonpolymeric polyol in the packaging solution will generally be an amount sufficient to form a more uniform coating on the surface of the lens when packaged in a packaging solution according to the present invention. In general, the concentration of the nonionic polyol will ordinarily range from about 0.01 to about 10 % w/w and preferably from about 0.1 to about 3.0 % w/w.

Without being bound by any particular theory, it is believed that the nonionic, nonpolymeric polyol molecules are able to penetrate into the matrix of the lens material, especially a hydrogel lens material, thus attracting and binding water molecules into the lens matrix. The polymeric conditioning agent forms a film around the lens, thus trapping and retaining the moisture and leading to a slow release of the polyol throughout the duration of wear. Thus, these two components work in combination for a unique effect. Accordingly, preferred polyols are humectants, which are substances that attract and retain moisture because of their ability to form hydrogen bonds with water molecules, and preferred polymeric conditioning agents are demulcents.

The packaging solutions according to the present invention are physiologically compatible. Specifically, the solution must be "ophthalmically safe" for use with a lens such as a contact lens, meaning that a contact lens treated with the solution is generally suitable and safe for direct placement on the eye without rinsing, that is, the solution is safe and comfortable for daily contact with the eye via a contact lens that has been wetted with the solution. An ophthalmically safe solution has a tonicity and pH that is compatible with the eye and includes materials, and amounts thereof, that are non-cytotoxic according to ISO standards and U.S. Food & Drug Administration (FDA) regulations. The solution should be sterile in that the absence of microbial contaminants in the product prior to release must be statistically demonstrated to the degree necessary for such products. The liquid media useful in the present invention are selected to have no substantial detrimental effect on the lens being treated or cared for and to allow or even facilitate the present lens treatment or treatments. The liquid media are preferably aqueous-based. A particularly useful aqueous liquid medium is that derived from saline, for example, a conventional saline solution or a conventional buffered saline solution.

The pH of the present solutions should be maintained within the range of about 6.0 to about 8, and preferably about 6.5 to about 7.8. Suitable buffers may be added, such as: phosphate; borate; citrate; carbonate; tris-(hydroxymethyl)aminomethane (TRIS); bis(2-hydroxyethyl)-imino-tris-(hydroxymethyl)aminoalcohol (bis-tris); zwitterionic buffers such as N-[2-Hydroxy-1,1-bis(hydroxymethyl)ethyl]glycine (Tricine)and N-[2-Hydroxy-1,1-bis(hydroxymethyl)ethyl]glycine, MOPS; N-(Carbamoylmethyl)taurine (ACES); amino acids and amino acid derivatives; and mixtures thereof. Generally, buffers will be used in amounts ranging from about 0.05 to about 2.5 percent by weight, and preferably from about 0.1 to about 1.5 percent by weight of the solution. The packaging solutions of this invention preferably contain a borate buffer, containing one or more of boric acid, sodium borate, potassium tetraborate, potassium metaborate or mixtures of the same.

If needed, the solutions of the present invention may be adjusted with tonicity agents, to approximate the osmotic pressure of normal lacrimal fluids, which is equivalent to a 0.9 percent solution of sodium chloride or 2.5 percent of glycerol solution. The solutions are made substantially isotonic with physiological saline used alone or in combination, otherwise if simply blended with sterile water and made hypotonic or made hypertonic the lenses will lose their desirable optical parameters. Correspondingly, excess saline may result in the formation of a hypertonic solution, which will cause stinging, and eye irritation.

Examples of suitable tonicity adjusting agents include, but are not limited to, sodium and potassium chloride, dextrose, calcium and magnesium chloride and the like and mixtures thereof. These agents are typically used individually in amounts ranging from about 0.01 to about 2.5% w/v and preferably from about 0.2 to about 1.5% w/v. Preferably, the tonicity agent will be employed in an amount to provide a final osmotic value of at least about 200 mOsm/kg, preferably from about 200 to about 450 mOsm/kg, more preferably from about 250 to about 400 mOsm/kg, and most preferably from about 280 to about 370 mOsm/kg.

If desired, one or more additional components can be included in the packaging solution. Such additional component or components are chosen to impart or provide at least one beneficial or desired property to the packaging solution. Such additional components may be selected from components that are conventionally used in one or more ophthalmic device care compositions. Examples of such additional components include cleaning agents, wetting agents, nutrient agents, sequestering agents, viscosity builders, contact lens conditioning agents, antioxidants, and the like and mixtures thereof. These additional components may each be included in the packaging solutions in an amount effective to impart or provide the beneficial or desired property to the packaging solutions. For example, such additional components may be included in the packaging solutions in amounts similar to the amounts of such components used in other, e.g., conventional, contact lens care products.

Useful sequestering agents include, but are not limited to, disodium ethylene diamine tetraacetate, alkali metal hexametaphosphate, citric acid, sodium citrate and the like and mixtures thereof.

Useful antioxidants include, but are not limited to, sodium metabisulfite, sodium thiosulfate, N-acetylcysteine, butylated hydroxyanisole, butylated hydroxytoluene and the like and mixtures thereof.

The method of packaging and storing an ophthalmic lens according to the present invention includes at least packaging the ophthalmic lens immersed in the aqueous contact lens packaging solution described above. The method may include immersing the ophthalmic lens in an aqueous contact lens solution prior to delivery to the customer/wearer, directly following manufacture of the contact lens. Alternately, the packaging and storing in the solution of the present invention may occur at an intermediate point before delivery to the ultimate customer (wearer) but following manufacture and transportation of the lens in a dry state, wherein the dry lens is hydrated by immersing the lens in the contact lens packaging solution. Consequently, a package for delivery to a customer may include a sealed container containing one or more unused contact lenses immersed in an aqueous contact lens packaging solution according to the present invention.

In one embodiment, the steps leading to the present ophthalmic device packaging system include (1) molding an ophthalmic device in a mold comprising at least a first and second mold portion, (2) removing the lens from the mold portions; (3) introducing the packing solution of this invention and the ophthalmic lens into the container, and (4) sealing the container. Preferably, the method also includes the step of sterilizing the contents of the container. Sterilization may take place prior to, or most conveniently after, sealing of the container and may be effected by any suitable method known in the art, e.g., by balanced autoclaving of the sealed container at temperatures of about 120°C or higher. Preferred packages are plastic blister packages, including a recess for receiving a contact lens and the package solution, where the recess is sealed with lidstock prior to sterilization of the package contents.

The following examples are provided to enable one skilled in the art to practice the invention and are merely illustrative of the invention. The examples should not be read as limiting the scope of the invention as defined in the claims.

Tables 1 to 3 illustrate various solutions of this invention. Additionally, the pH of the solutions in Tables 2 and 3 was adjusted with NaOH/HCl

**Table 1**

| Compound | Preferred Concentration Range %w/v | Concentration %w/v |
|---|---|---|
| NaH₂PO₄ | 0.05-1.0 | 0.3 |
| Na₂HPO₄ | 0.05-0.5 | 0.15 |
| NaCl | 0.01-1.0 | 0.6 |
| Sorbitol | 0.1-2.0 | 0.5 |
| HPMC | 0.05-2.0 | 0.15 |
| water | Q.S. to 100% w/v | Q.S. to 100% w/v |

**Table 2**

| Compound | Preferred Concentration Range %w/v | Concentration %w/v |
|---|---|---|
| MOPS | 0.1-3.0 | 2.0 |
| NaCl | 0.01-1.0 | 0.5 |
| Glycerin | 0.1-2.0 | 0.5 |
| Tetronic 1107 | 0.05-2.0 | 0.5 |
| water | Q.S. to 100% w/v | Q. S. to 100% w/v |

| | | |
|---|---|---|
| * pH adjusted with NaOH/HCl | | |

**Table 3**

| Compound | Preferred Concentration Range %w/v | Concentration %w/v |
|---|---|---|
| Sodium citrate | 0.1-3.0 | 0.65 |
| KCl | 0.01-1.0 | 0.10 |
| Mannitol | 0.1-2.0 | 1.0 |
| PVA | 0.05-2.0 | 0.5 |
| water | Q.S. to 100% w/v | Q.S. to 100% w/v |

| | | |
|---|---|---|
| * pH adjusted with NaOH/HCl | | |

Tables 4 and 5 list other solutions of this invention, as well as two comparative solutions.

**Table 4**

| **Comparative A** | **Invention B** |
|---|---|
| 0.90 %w/v Boric Acid | 0.90 %w/v Boric Acid |
| 0.10 %w/v Sodium Borate | 0.10 %w/v Sodium Borate |
| 0.60 %w/v NaCl | 0.42 %w/v NaCl |
| Q.S to 100% w/v with water | 0.50 %w/v Glycerin |
| | 0.50 %w/v Tetronic 1107 |
| | Q. S to 100% w/v with water |

**Table 5**

| **Invention C** | **Comparative D** |
|---|---|
| 0.12 %w/v NaH₂PO₄ | 0.12 %w/v NaH₂PO₄ |
| 0.30 %w/v Na₂HPO₄ | 0.30 %w/v Na₂HPO₄ |
| 0.60 %w/v NaCl | 0.88 %w/v NaCl |
| 0.50 %w/v Glycerin | 0.5 %w/v Polyvinyl alcohol |
| 0.5 %w/v Polyvinyl alcohol | Q.S to 100% w/v with water |
| Q.S to 100% w/v with water | |

The solutions in Tables 4 and 5 were tested as follows. Contact lenses were soaked in the respective solutions of Tables 4 and 5 for no fewer than 72 hours. Lenses were then removed from the test solution and were immediately mounted and tested in 1 mL phosphate borate saline (PBS). Tribological testing was performed on a CETR Model UMT-2 micro-tribometer. Each lens was clamped on an HDPE holder that initially mates with the posterior side of the lens. A poly(propylene) clamping ring was then used to hold the edge region of the lens. Once the lens was mounted in the holder the assembly was placed in a stationary clamping device within the micro-tribometer. A polished stainless steel disc containing 1 mL of the test solution was then brought into contact with the lens and F_{N} was adjusted to 2 grams over the course of the run for the frictional measurements. After the load equilibrated for 5 seconds the stainless steel disc was rotated at a velocity of 12cm/sec for a duration of 20 sec in both the forward and reverse directions and the peak (static) and average (kinetic) COF values were recorded. Each value represents the average of 9-10 lenses. Controls were run from the same lot of lenses that were not subjected to any test solution. All data was normalized to the average values obtained from the lens holder in the absence of a lens tested in PBS. The results are summarized in Table 6.

**Table 6**

| Solution | Mean static COF | Standard error | Mean kinetic COF | Standard error |
|---|---|---|---|---|
| A | 2.657 | 0.157 | 0.2810 | 0.0109 |
| B | 1.737 | 0.157 | 0.1379 | 0.0109 |
| C | 1.265 | 0.157 | 0.2755 | 0.0109 |
| D | 1.867 | 0.157 | 0.2866 | 0.0115 |

Tribology is the study of how two surfaces interact with each other when in relative motion. One aspect of tribology that may be of importance to contact lenses is friction. Friction is a measure of a material's resistance to lateral motion when placed against a specific substrate. The relative friction between two surfaces may be described in terms of a coefficient of friction (COF), which is defined as the ratio of the lateral force (Fₓ) that is required to initiate and then sustain movement to the normal force (F_{N}). Further, there are two friction coefficients that may be considered, the peak (or static) and average (or kinetic). The static COF is a measure of how much Fₓ is needed to initiate relative motion of two surfaces and is typically the larger of the two values. Practically, for contact lenses, the static COF is related to the amount of force needed to start a blink cycle or for the lens to begin moving over the cornea. The kinetic COF is a measure of how much lateral force is needed to sustain movement at a particular velocity averaged over a finite period of time. This value is related to the amount of force required to sustain the blink over the course of the entire cycle and the ease of motion of the lens on the cornea (which may be further related to how much the lens moves on the cornea).

It will be understood that various modifications may be made to the embodiments disclosed herein. Therefore the above description should not be construed as limiting, but merely as exemplifications of preferred embodiments. For example, the functions described above and implemented as the best mode for operating the present invention are for illustration purposes only. Other arrangements and methods may be implemented by those skilled in the art without departing from the scope and spirit of this invention. Moreover, those skilled in the art will envision other modifications within the scope of the claims appended hereto.

## Claims

1. A method of preparing a package comprising a storable, sterile ophthalmic device, the method comprising:
(a) immersing an ophthalmic device in a solution comprising a nonionic, nonpolymeric polyol and a nonionic, polymeric conditioning agent, wherein the solution has an osmolality of at least about 200 mOsm/kg and a pH in the range of about 6 to about 8, and wherein the concentration of the nonionic, polymeric conditioning agent is about 0.01 to about 10 % w/w;
(b) packaging the solution and the device in a manner preventing contamination of the device by microorganisms; and
(c) sterilizing the packaged solution and device.

2. The method of Claim 1, wherein the ophthalmic device is a contact lens.

3. The method of Claim 1, wherein the ophthalmic device comprises a polymerization product of a monomeric mixture comprising one or more silicone- containing monomers.

4. The method of Claim 1, wherein the nonionic, polymeric conditioning agent comprises at least one member selected from the group consisting of nonionic polysaccharide, polyvinylalcohol, polyvinylpyrrolidone, and a polymer of PEO.

5. The method of Claim 4, wherein the nonionic, polymeric conditioning agent comprises at least one member selected from the group consisting of methylcellulose; hydroxyethylcellulose; hydroxypropyl cellulose, hydroxypropylmethylcellulose; and methylhydroxyethyl starch.

6. The method of Claim 4, wherein the nonionic, polymeric conditioning agent comprises a block copolymers of PEO and PPO.

7. The method of Claim 4, wherein the nonionic, polymeric conditioning agent comprises polyvinylalcohol.

8. The method of Claim 1, wherein the nonionic, nonpolymeric polyol contains 2 to 12 carbon atoms and 2 to 8 hydroxyl groups.

9. The method of Claim 1, wherein the nonionic, nonpolymeric polyol includes at least one member selected from the group consisting of glycerin, ethylene glycol, propylene glycol, sorbitol, manitol, monosaccarides, and disaccharides.

10. The method of Claim 9, wherein the wherein the nonionic, nonpolymeric polyol includes at least one member selected from the group consisting of glycerin, sorbitol and manitol.

11. The method of Claim 1, wherein the solution further comprises a buffering agent.

12. The method of Claim 11, wherein the buffering agent comprises at least one member selected from the group consisting of: phosphate; borate; citrate; carbonate; TRIS; bis-tris; Tricine; MOPS; ACES; and an amino acid.

13. The method of Claim 1, including hermetically sealing the contact lens and the contact lens packing solution in the package and heat sterilizing the package contents.

14. The method of Claim 1, wherein the solution does not contain an effective disinfecting amount of a disinfecting agent.

15. The method of Claim 1, wherein the solution does not contain a germicide compound.

16. A packaging system for the storage of an ophthalmic device comprising a sealed container containing an unused ophthalmic device immersed in an aqueous packing solution comprising a nonionic, nonpolymeric polyol and a nonionic, polymeric conditioning agent, wherein the solution has an osmolality of at least about 200 mOsm/kg and a pH in the range of about 6 to about 8, and wherein the concentration of the nonionic, polymeric conditioning agent is about 0.01 to about 10 % w/w.

17. The packaging system of Claim 16, wherein the ophthalmic device is a contact lens.

18. The packaging system of Claim 17, wherein the ophthalmic device comprises a silicone hydrogel contact lens.

19. The packaging system of Claim 17, wherein the solution does not contain an effective disinfecting amount of a disinfecting agent.

## Patentansprüche

1. Ein Verfahren zur Herstellung einer Verpackung, welche eine lagerfähige, sterile ophthalmische Vorrichtung umfasst, wobei das Verfahren umfasst:
(a) Eintauchen einer ophthalmischen Vorrichtung in eine Lösung, welche ein nichtionisches, nichtpolymeres Polyol und ein nichtionisches , polymeres Konditionierungsmittel umfasst, wobei die Lösung eine Osmolalität von wenigstens etwa 200 mOsm/kg und einen pH-Wert im Bereich zwischen etwa 6 und etwa 8 aufweist und wobei die Konzentration des nichtionischen, polymeren Konditionierungsmittels etwa 0.01 bis etwa 10 % w/w beträgt;
(b) Verpacken der Lösung und der Vorrichtung auf eine Weise, welche die Kontamination der Vorrichtung durch Mikroorganismen verhindert; und
(c) Sterilisieren der verpackten Lösung und Vorrichtung.

2. Das Verfahren gemäß Anspruch 1, wobei die ophthalmische Vorrichtung eine Kontaktlinse ist.

3. Das Verfahren gemäß Anspruch 1, wobei die ophthalmische Vorrichtung ein Polymerisationsprodukt einer monomeren Mischung umfasst, welche ein oder mehrere silikonhaltige Monomere umfasst.

4. Das Verfahren gemäß Anspruch 1, wobei das nichtionische, polymere Konditionierungsmittel wenigstens eine Komponente ausgewählt aus der Gruppe bestehend aus nichtionischem Polysaccharid, Polyvinylalkohol, Polyvinylpyrrolidon und einem Polymer aus PEO, umfasst.

5. Das Verfahren gemäß Anspruch 4, wobei das nichtionische, polymere Konditionierungsmittel wenigstens eine Komponente ausgewählt aus der Gruppe bestehend aus Methylcellulose; Hydroxyethylcellulose; Hydroxypropylcellulose, Hydroxypropylmethylcellulose und Methylhydroxyethylstärke umfasst.

6. Das Verfahren gemäß Anspruch 4, wobei das nichtionische, polymere Konditionierungsmittel ein Block-Copolymer aus PEO und PPO umfasst.

7. Das Verfahren gemäß Anspruch 4, wobei das nichtionische, polymere Konditionierungsmittel Polyvinylalkohol umfasst.

8. Das Verfahren gemäß Anspruch 1, wobei das nichtionische, nichtpolymere Polyol 2 bis 12 Kohlenstoffatome und 2 bis 8 Hydroxylgruppen enthält.

9. Das Verfahren gemäß Anspruch 1, wobei das nichtionische, nichtpolymere Polyol wenigstens eine Komponente ausgewählt aus der Gruppe bestehend aus Glycerin, Ethylenglykol, Propylenglykol, Sorbitol, Mannitol, Monosacchariden und Disacchariden beinhaltet.

10. Das Verfahren gemäß Anspruch 9, wobei das nichtionische, nichtpolymere Polyol wenigstens eine Komponente ausgewählt aus der Gruppe bestehend aus Glycerin, Sorbitol und Mannitol beinhaltet.

11. Das Verfahren gemäß Anspruch 1, wobei die Lösung weiterhin ein Puffermittel umfasst.

12. Das Verfahren gemäß Anspruch 11, wobei das Puffermittel wenigstens eine Komponente ausgewählt aus der Gruppe bestehend aus Phosphat, Borat, Citrat, Carbonat, TRIS, Bis-Tris, Tricin, MOPS, ACES und einer Aminosäure umfasst.

13. Das Verfahren gemäß Anspruch 1, welches das hermetische Verschließen der Kontaktlinse und der Kontaktlinsenverpackungslösung in der Verpackung und das Hitzesterilisieren des Packguts beinhaltet.

14. Das Verfahren gemäß Anspruch 1, wobei die Lösung keine wirksam desinfizierende Menge eines Desinfektionsmittels enthält.

15. Das Verfahren gemäß Anspruch 1, wobei die Lösung keine Germizidverbindung enthält.

16. Ein Verpackungssystem zur Lagerung einer ophthalmischen Vorrichtung, welches einen verschlossenen Behälter umfasst, der eine ungebrauchte ophthalmische Vorrichtung eingetaucht in eine wässrige Verpackungslösung, welche ein nichtionisches, nichtpolymeres Polyol und ein nichtionisches , polymeres Konditionierungsmittel umfasst, wobei die Lösung eine Osmolalität von wenigstens etwa 200 mOsm/kg und einen pH-Wert im Bereich von etwa 6 bis etwa 8 aufweist und wobei die Konzentration des nichtionischen, polymeren Konditionierungsmittels etwa 0.01 bis etwa 10 % w/w beträgt, enthält.

17. Das Verpackungssystem gemäß Anspruch 16, wobei die ophthalmische Vorrichtung eine Kontaktlinse ist.

18. Das Verpackungssystem gemäß Anspruch 17, wobei die ophthalische Vorrichtung eine Silikon-Hydrogel-Kontaktlinse umfasst.

19. Das Verpackungssystem gemäß Anspruch 17, wobei die Lösung keine wirksam desinfizierende Menge eines Desinfektionsmittels enthält.

## Revendications

1. Procédé de préparation d'un conditionnement comprenant un dispositif ophtalmique stérile stockable, lequel procédé comprend :
(a) l'immersion d'un dispositif ophtalmique dans une solution comprenant un polyol non polymère non-ionique et un agent conditionnant polymère non-ionique, la solution ayant une osmolalité d'au moins environ 200 mOsm/kg et un pH situé dans la plage allant d'environ 6 à environ 8, et la concentration de l'agent conditionnant polymère non-ionique étant d'environ 0,01 à environ 10 % p/p ;
(b) conditionner la solution et le dispositif d'une manière empêchant la contamination du dispositif par des micro-organismes ; et
(c) stériliser la solution et le dispositif conditionnés.

2. Procédé selon la revendication 1, dans lequel le dispositif ophtalmique est une lentille de contact.

3. Procédé selon la revendication 1, dans lequel le dispositif ophtalmique comprend un produit de polymérisation d'un mélange de monomères comprenant un ou plusieurs monomères siliconés.

4. Procédé selon la revendication 1, dans lequel l'agent conditionnant polymère non-ionique comprend au moins un membre choisi dans le groupe constitué par un polysaccharide non-ionique, le poly(alcool vinylique), la polyvinylpyrrolidone, et un polymère de PEO.

5. Procédé selon la revendication 4, dans lequel l'agent conditionnant polymère non-ionique comprend au moins un membre choisi dans le groupe constitué par la méthylcellulose ; l'hydroxyéthylcellulose ; l'hydroxy-propylcellulose, l'hydroxypropylméthylcellulose ; et le méthylhydroxyéthyl-amidon.

6. Procédé selon la revendication 4, dans lequel l'agent conditionnant polymère non-ionique comprend un copolymère séquencé de PEO et PPO.

7. Procédé selon la revendication 4, dans lequel l'agent conditionnant polymère non-ionique comprend du poly(alcool vinylique).

8. Procédé selon la revendication 1, dans lequel le polyol non polymère non-ionique contient 2 à 12 atomes de carbone et 2 à 8 groupes hydroxyle.

9. Procédé selon la revendication 1, dans lequel le polyol non polymère non-ionique englobe au moins un membre choisi dans le groupe constitué par le glycérol, l'éthylèneglycol, le propylèneglycol, le sorbitol, le mannitol, les monosaccharides, et les disaccharides.

10. Procédé selon la revendication 9, dans lequel le polyol non polymère non-ionique englobe au moins un membre choisi dans le groupe constitué par le glycérol, le sorbitol et le mannitol.

11. Procédé selon la revendication 1, dans lequel la solution comprend en outre un agent tampon.

12. Procédé selon la revendication 11, dans lequel l'agent tampon comprend au moins un membre choisi dans le groupe constitué par : un phosphate ; borate ; citrate ; carbonate ; TRIS ; bis-tris ; Tricine ; MOPS ; ACES ; et un acide aminé.

13. Procédé selon la revendication 1, comprenant le scellage hermétique de la lentille de contact et de la solution de conditionnement de lentille de contact dans le conditionnement et la stérilisation à la chaleur des contenus du conditionnement.

14. Procédé selon la revendication 1, dans lequel la solution ne contient pas une quantité désinfectante efficace d'un agent désinfectant.

15. Procédé selon la revendication 1, dans lequel la solution ne contient pas de composé germicide.

16. Système de conditionnement pour le stockage d'un dispositif ophtalmique comprenant un récipient scellé contenant un dispositif ophtalmique non utilisé immergé dans une solution de conditionnement aqueuse comprenant un polyol non polymère non-ionique et un agent conditionnant polymère non-ionique, dans lequel la solution a une osmolalité d'au moins environ 200 mOsm/kg et un pH situé dans la plage allant d'environ 6 à environ 8, et dans lequel la concentration de l'agent conditionnant polymère non-ionique est d'environ 0,01 à environ 10 % p/p.

17. Système de conditionnement selon la revendication 16, dans lequel le dispositif ophtalmique est une lentille de contact.

18. Système de conditionnement selon la revendication 17, dans lequel le dispositif ophtalmique comprend une lentille de contact en hydrogel de silicone.

19. Système de conditionnement selon la revendication 17, dans lequel la solution ne contient pas une quantité désinfectante efficace d'un agent désinfectant.
